# EUROPÄISCHE PATENTANMELDUNG

(11) **EP 1 875 891 A1**
(43) Veröffentlichungstag der Anmeldung: **09.01.2008**
(21) Anmeldenummer: 07012870.7
(22) Anmeldetag: 30.06.2007
(51) Int. Cl.: A61K 8/44, A61K 8/46, A61Q 5/02, A61Q 19/10

(54) **Kosmetische oder dermatologische Reinigungsmittel enthaltend sekundäre Alkansulfonate**

(30) Priorität: 06.07.2006 DE 102006031377
(71) Anmelder: Clariant International Ltd., 4132 Muttenz (CH)
(72) Erfinder: Klug, Peter, Dr., 63762 Grossostheim (DE); Simsch, Waltraud, 65779 Kelkheim (DE); Mulitze-Kleinheyer, Vera, 65929 Frankfurt am Main (DE)
(74) Vertreter: Paczkowski, Marcus

(57) **Zusammenfassung**

Es werden stabile flüssige kosmetische oder dermatologische Reinigungsmittel enthaltend ein oder mehrere sekundäre Alkansulfonate und ein oder mehrere Betaine beschrieben, die dadurch gekennzeichnet sind, dass das Gewichtsverhältnis von Alkansulfonat zu Betain von 6 : 3 bis 1: 2 ist und die Viskosität des Reinigungsmittels zwischen 1000 und 30000 mPas beträgt.

## Beschreibung

Die vorliegende Erfindung betrifft kosmetische, pharmazeutische und dermatologische Mittel enthaltend sekundäre Alkansulfonate und Betaine.

Verbraucherwünsche, dermatologische und toxikologische Aspekte, sowie die Rheologie kosmetischer Produkte sind eng miteinander verknüpft. So werden hautfreundliche, in toxikologischer und ökotoxikologischer Sicht unbedenkliche Produkte mit günstigen rheologischen Eigenschaften gefordert.

Sekundäre Alkansulfonate sind seit langem als Basistenside bekannt, speziell für Waschmittelanwendungen und industrielle Reiniger.

In US 2004/0 204 336 werden wässrige flüssige Dispersionen offenbart, enthaltend sekundäre Alkansulfonate, Builder und Elektrolyte, stabilisiert durch ein Alkylhydroxyethylammoniumsalz.

In US 2004/0 029 757 werden Handgeschirrspülmittelformulierungen beschrieben, wobei die Tensidmischung aus sekundären Alkansulfonaten, Alkylethersulfaten, Alkylsulfaten, Alkylmethylestersulfaten, α-Olefinsulfonaten und einem Betain besteht.

DE 101 62 648 offenbart sprühbare flüssige wässrige Reinigungsmittel, enthaltend eine Tensidkombination aus Alkylethersulfat, sekundärem Alkansulfonat und Amphotensid.

In WO 2006/050 875 werden Haarbehandlungsmittel beschrieben, die Alkylethersulfat oder Alkylsulfat oder deren Mischung, sekundäres Alkansulfonat, Betain oder Ethercarboxylat oder deren Mischung, ein oder mehrere nichtionische Tenside und ein kationisches Polymer enthalten.

In kosmetischen und pharmazeutischen Produkten wurde sekundäres Alkansulfonat in der Vergangenheit nur wenig eingesetzt.

Sekundäre Alkansulfonate haben im Gegensatz zu Alkylethersulfaten, beispielsweise Natriumlaurylethersulfat, oder Alkylsulfaten, beispielsweise Natriumlaurylsulfat, den Nachteil, dass sie auch in Gegenwart von Cotensiden wie z.B. Betainen nicht auf die üblichen Methoden der Verdickung, speziell auf die Zugabe von Kochsalz mit einer Viskositätszunahme reagieren. Dies gilt speziell bei den üblichen Verhältnissen von Basistensid zu Betain von 7 zu 3 oder 8 zu 2 und Gesamttensidgehalten von unter 20 Gew.- % in der Formulierung.

Es bestand die Aufgabe, Tensidsysteme für flüssige kosmetische Formulierungen, insbesondere zum Reinigen von Haut und Haaren, bereit zu stellen, die auf Viskositäten im Bereich von 1000 bis 30000 mPas auch bei Tensidkonzentrationen unterhalb 20 Gew.-% einstellbar sind und gleichzeitig hautfreundlich und toxikologisch unbedenklich sind.

Überraschenderweise wurde gefunden, dass bei Kombination von sekundären Alkansulfonaten mit Betainen sehr wohl viskose Tensidsysteme erhalten werden können, wenn das Gewichtsverhältnis von sekundärem Alkansulfonat zu Betain im Bereich von 6 zu 3 bis 1 zu 2 gewählt wird. Diese Systeme sind insbesondere bei pH-Werten von unter 6 sehr gut handhabbar und bilden farblose, klare und stabile Tensidlösungen, die als kosmetische und pharmazeutische Produkte in Form von Shampoos, Duschgelen oder Flüssigseifen Verwendung finden können.

Gegenstand der Erfindung sind stabile flüssige kosmetische oder dermatologische Reinigungsmittel enthaltend ein oder mehrere sekundäre Alkansulfonate und ein oder mehrere Betaine, dadurch gekennzeichnet, dass das Gewichtsverhältnis von Alkansulfonat zu Betain von 6 : 3 bis 1: 2 ist und die Viskosität des Reinigungsmittels zwischen 1000 und 30000 mPas beträgt.

Stabile Reinigungsmittel im Rahmen der vorliegenden Erfindung sind Formulierungen, die bei Temperaturen im Bereich von 0 bis +50°C und auch bei Lagerzeiten über mehrere Monate hinweg keine Separation, Ausfällungen und Trübungen zeigen.
Die Viskositäten wurden mittels eines Brookfield Viskosimeters Modell DV-II mit der Spindel Nr. 3 bei einer Rotationsgeschwindigkeit von 20 1/s ermittelt.

Die erfindungsgemäßen Reinigungsmittel zeichnen sich durch ein gutes Reinigungsvermögen, eine gute Hautmilde und ein ästhetisches Erscheinungsbild aus.

Das Gewichtsverhältnis von Alkansulfonat zu Betain ist in den erfindungsgemäßen Reinigungsmitteln vorzugsweise von 4 : 3 bis 1 : 1.

Die Viskosität beträgt in den erfindungsgemäßen Reinigungsmitteln vorzugsweise zwischen 2000 und 10000 mPas.

Die Menge an dem einen oder den mehreren sekundären Alkansulfonaten und dem einen oder den mehreren Betainen in den erfindungsgemäßen Reinigungsmitteln beträgt zusammen, bezogen auf das Gesamtgewicht der Mittel, vorzugsweise zwischen 6 und 20 Gew.-% und besonders bevorzugt zwischen 8 und 15 Gew.-%.

In einer bevorzugten Ausführungsform der Erfindung ist das eine oder sind die mehreren Betaine ausgewählt aus Alkylamidopropylbetainen gemäß Formel (1) 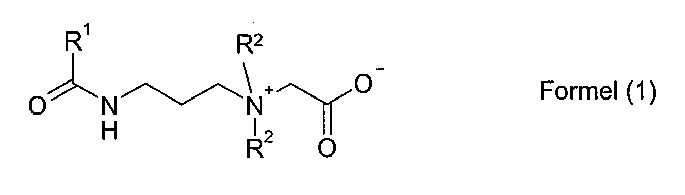 und Alkylbetainen gemäß Formel (2) 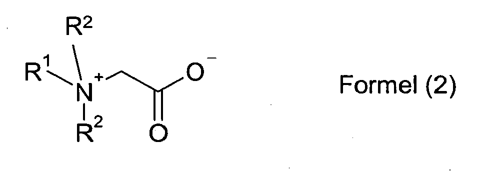 worin R¹ eine Alkyl-, Hydroxyalkyl- oder Alkylphenylgruppe, bevorzugt eine Alkylgruppe, mit 8 bis 22 Kohlenstoffatomen ist und jeder Rest R² für eine Methylgruppe steht.

In einer weiteren bevorzugten Ausführungsform der Erfindung ist das eine oder sind die mehreren Betaine ausgewählt aus der Gruppe bestehend aus Alkylamidopropylbetainen mit 8 bis 18 Kohlenstoffatomen in der Alkylgruppe des Fettsäurerests, Alkylbetainen mit 8 bis 18 Kohlenstoffen in der Alkylgruppe und Mischungen aus diesen Substanzen.

In einer besonders bevorzugten Ausführungsform der Erfindung ist das eine oder sind die mehreren Betaine ausgewählt aus Alkylamidopropylbetain oder einer Mischung aus Alkylamidopropylbetain und Alkylbetain.

In einer weiteren bevorzugten Ausführungsform der Erfindung sind die Reinigungsmittel klar.

In den erfindungsgemäßen Reinigungsmitteln werden sekundäre Alkansulfonate eingesetzt, deren Alkylgruppe gesättigt oder ungesättigt, linear oder verzweigt ist und die optional auch Hydroxylgruppen tragen können, wobei die endständigen Kohlenstoffatome der Alkylkette keine Sulfonatgruppe aufweisen.

Bevorzugt sind sekundäre Alkansulfonate mit linearen Alkylgruppen mit 8 bis 20 Kohlenstoffatomen, die statistisch verteilt eine oder mehrere SO₃X-Gruppen in Sekundärstellung an der Kohlenwasserstoffkette tragen. Das Gegenion X kann ein Natrium-, Kalium-, Ammonium-, Mono-, Di- oder Triethanolammonium, Calcium-, oder Magnesiumion sein oder eine Mischung aus den genannten Gegenionen. Bevorzugt sind Natriumsalze der sekundären Alkansulfonate.

In einer weiteren bevorzugten Ausführungsform der Erfindung ist das eine oder sind die mehreren sekundären Alkansulfonate ausgewählt aus C₈₋₂₀ Alkansulfonaten.

In einer besonders bevorzugten Ausführungsform der Erfindung ist das eine oder sind die mehreren sekundären Alkansulfonate ausgewählt aus C₁₄₋₁₇ Alkansulfonaten, vorzugsweise aus deren Natriumsalzen.

Alkoxylierte Tenside, insbesondere Alkylethersulfate enthalten herstellungsbedingt in geringeren Mengen kurzkettige Glykole, die als toxikologisch bedenklich bewertet werden, so dass im Markt eine Nachfrage nach sogenannten EO-freien, d.h. Ethylenoxid-freien, oder sogenannten ethersulfatfreien Formulierungen besteht.

Die erfindungsgemäßen Reinigungsmittel zeichnen sich dadurch aus, dass sie keine ethoxylierten oder propoxylierten Verbindungen enthalten müssen. In einer weiteren bevorzugten Ausführungsform der Erfindung enthalten die Reinigungsmittel daher keine ethoxylierten oder propoxylierten Verbindungen. In einer besonders bevorzugten Ausführungsform der Erfindung enthalten die Reinigungsmittel keine Alkylethersulfate.

In einer weiteren bevorzugten Ausführungsform der Erfindung enthalten die Reinigungsmittel neben dem einen oder den mehreren sekundären Alkansulfonaten und dem einen oder den mehreren Betainen ein oder mehrere Cotenside, vorzugsweise ausgewählt aus der Gruppe der anionischen, kationischen, nichtionischen, zwitter-ionischen und amphoteren Tenside.

Als zusätzliche anionische Tenside eignen sich bevorzugt (C₁₀-C₂₀)-Alkyl- und Alkylen-carboxylate, Alkylethercarboxylate, Fettalkoholsulfate, Fettalkoholethersulfate, Alkylamidsulfate und -sulfonate, Fettsäurealkylamidpolyglykolethersulfate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und - diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Acylglutamate.

Die Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie der analogen Alkylammonium-Salze.

Der Gewichtsanteil der zusätzlichen anionischen Tenside ist, bezogen auf das Gesamtgewicht der Mittel, vorzugsweise von 0,5 bis 10 Gew.-%, besonders bevorzugt von 1 bis 8 Gew.-% und insbesondere bevorzugt von 2 bis 5 Gew.-%.

Geeignete kationische Tenside sind beispielsweise quartäre Ammoniumsalze wie Di-(C₁₀-C₂₄)-Alkyl-dimethylammoniumchlorid oder-bromid, vorzugsweise Di-(C₁₂-C₁₈)-Alkyl-dimethylammoniumchlorid oder -bromid; (C₁₀-C₂₄)-Alkyl-dimethylethylammoniumchlorid oder-bromid; (C₁₀-C₂₄)-Alkyl-trimethylammoniumchlorid oder-bromid, vorzugsweise Cetyltrimethylammoniumchlorid oder -bromid und (C₂₀-C₂₂)-Alkyl-trimethylammoniumchlorid oder -bromid; (C₁₀-C₂₄)-Alkyl-dimethylbenzylammoniumchlorid oder-bromid, vorzugsweise (C₁₂-C₁₈)-Alkyl-dimethylbenzylammoniumchlorid; N-(C₁₀-C₁₈)-Alkyl-pyridiniumchlorid oder-bromid, vorzugsweise N-(C₁₂-C₁₆)-Alkyl-pyridiniumchlorid oder -bromid; N-(C₁₀-C₁₈)-Alkyl-isochinoliniumchlorid, -bromid oder -monoalkylsulfat; N-(C₁₂-C₁₈)-Alkyl-polyoylaminoformylmethylpyridiniumchlorid; N-(C₁₂-C₁₈)-Alkyl-N-methyl-morpholinium-chlorid, -bromid oder -monoalkylsulfat; N-(C₁₂-C₁₈)-Alkyl-N-ethyl-morpholinium-chlorid, -bromid oder -monoalkylsulfat; (C₁₆-C₁₈)-Alkyl-pentaoxethyl-ammonium-chlorid; Diisobutylphenoxyethoxyethyldimethylbenzylammonium-chlorid; Salze des N,N-Diethylaminoethylstearylamids und -oleylamids mit Salzsäure, Essigsäure, Milchsäure, Zitronensäure, Phosphorsäure; N-Acyl-aminoethyl-N,N-diethyl-N-methylammoniumchlorid, -bromid oder -monoalkylsulfat und N-Acylaminoethyl-N,N-diethyl-N-benzyl-ammonium-chlorid, -bromid oder -monoalkylsulfat, wobei Acyl vorzugsweise für Stearyl oder Oleyl steht.

Der Gewichtsanteil der kationischen Tenside ist, bezogen auf das Gesamtgewicht der Mittel, vorzugsweise von 0,5 bis 10 Gew.-% und besonders bevorzugt von 1 bis 5 Gew.-%.

Als nichtionische Tenside eignen sich bevorzugt N-Alkyl-, N-Alkoxypolyhydroxyfettsäureamid, Fettsäure-N-alkylglucamide, Alkylpolyglucosid, Alkylphenolpolyethylenglykole; Alkylmercaptanpolyethylenglykole, Fettalkoholethoxylate, Fettaminethoxylate (Alkylaminopolyethylenglykole), Polypropylenglykolethoxylate (Pluronics^{®}), Saccharoseester; Sorbitester und Polyglykolether.

Der Gewichtsanteil der nichtionischen Tenside ist, bezogen auf das Gesamtgewicht der Mittel, vorzugsweise von 0,5 bis 10 Gew.-%, besonders bevorzugt von 1 bis 5 Gew.-% und insbesondere bevorzugt von 2 bis 4 Gew.-%.

Bevorzugte Amphotenside sind Acylglutamate, Amphoacetate, N-(C₁₂-C₁₈)-Alkyl-β-aminopropionate und N-(C₁₂-C₁₈)-Alkyl-β-iminodipropionate als Alkali- und Mono-, Di-und Trialkylammonium-Salze; Amphotenside auf Basis Imidazolin (Handelsname: Miranol^{®}, Steinapon^{®}), vorzugsweise das Natrium-Salz des 1-(β-Carboxymethyloxyethyl)-1-(carboxymethyl)-2-lauryl-imidazoliniums; Aminoxid, z.B. (C₁₂-C₁₈)-Alkyl-dimethylaminoxid, Fettsäureamidoalkyl-dimethylaminoxid.

Der Gewichtsanteil der zusätzlichen amphoteren Tenside ist, bezogen auf das Gsamtgewicht der Mittel, vorzugsweise von 0,5 bis 10 Gew.-% und besonders bevorzugt von 1 bis 8 Gew.-%.

Besonders bevorzugte erfindungsgemäße Reinigungsmittel enthalten ein oder mehrere Cotenside, die ausgewählt sind aus Acylglutamaten, Alkylpolyglucosiden, Amphoacetaten, Cocosmonoethanolamiden, Cocosisopropanolamiden oder Mischungen aus diesen Substanzen.

Die Gesamtmenge der eingesetzten Tenside, also die Summe aus sekundären Alkansulfonaten, Betainen und Cotensiden beträgt, insofern letztere in dem Mittel enthalten sind und bezogen auf das Gesamtgewicht des Mittels, vorzugsweise von 6,5 bis 20 Gew.-%, besonders bevorzugt von 8 bis 18 Gew.-% und insbesondere bevorzugt von 10 bis 16 Gew.-%.

Die erfindungsgemäßen Mittel können als weitere Hilfs- und Zusatzstoffe Ölkörper, Emulgatoren und Co-Emulgatoren, sowie weitere im kosmetischen, pharmazeutischen und dermatologischen Bereich gebräuchliche Zusätze, wie z.B. kationische Polymere, Filmbildner, Überfettungsmittel, Stabilisatoren, biogene Wirkstoffe, Glycerin, Konservierungsmittel, Perlglanzmittel, Farb- und Duftstoffe, Lösungsmittel, Trübungsmittel, ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen enthalten. Des Weiteren können den erfindungsgemäßen Mitteln antimikrobiell wirkende Agentien zugesetzt werden.

Unter Ölkörper ist jegliche Fettsubstanz zu verstehen, die bei Raumtemperatur (25°C) flüssig ist.

Die Fett-Phase kann daher ein oder mehrere Öle umfassen, die vorzugsweise aus folgenden Ölen ausgewählt werden:

Silikonöle, flüchtig oder nicht flüchtig, linear, verzweigt oder ringförmig, eventuell organisch modifiziert; Phenylsilikone; Silikonharze und -gummis; Mineralöle wie Paraffin- oder Vaselinöl; Öle tierischen Ursprungs wie Perhydrosqualen, Lanolin; Öle pflanzlichen Ursprungs wie flüssige Triglyceride, z.B. Sonnenblumen-, Mais-, Soja-, Reis-, Jojoba-, Babusscu-, Kürbis-, Traubenkern-, Sesam-, Walnuss-, Aprikosen-, Makadamia-, Avocado-, Süßmandel-, Wiesenschaumkraut-, Ricinusöl, Triglyceride der Capryl/Caprinsäuren, Olivenöl, Erdnussöl, Rapsöl und Kokosnussöl; Synthetische Öle wie Purcellinöl, Isoparaffine, lineare und/oder verzweigte Fettalkohole und Fettsäureester, bevorzugt Guerbetalkohole mit 6 bis 18, vorzugsweise 8 bis 10, Kohlenstoffatomen; Ester von linearen (C₆-C₁₃)-Fettsäuren mit linearen (C₆-C₂₀)-Fettalkoholen; Ester von verzweigten (C₆-C₁₃)-Carbonsäuren mit linearen (C₆-C₂₀)-Fettalkoholen, Ester von linearen (C₆-C₁₈)-Fettsäuren mit verzweigten Alkoholen, insbesondere 2-Ethylhexanol; Ester von linearen und/oder verzweigten Fettsäuren mit mehrwertigen Alkoholen (wie z.B. Dimerdiol oder Trimerdiol) und/oder Guerbetalkoholen; Triglyceride auf Basis (C₆-C₁₀)-Fettsäuren; Ester wie Dioctyladipat, Diisopropyl dimer dilinoleat; Propylenglykole/-dicaprylat oder Wachse wie Bienenwachs, Paraffinwachs oder Mikrowachse, gegebenenfalls in Kombination mit hydrophilen Wachsen, wie z.B. Cetylstearylalkohol; fluorierte und perfluorierte Öle; fluorierte Silikonöle; Gemische der vorgenannten Verbindungen.

Als kationische Polymere eignen sich die unter der INCI-Bezeichnung "Polyquaternium" bekannten Substanzen, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVP-dimethylaminoethylmethacrylat-Copolymer, Guarhydroxypropyltriammoniumchloride, sowie Calciumalginat und Ammoniumalginat. Des weiteren können eingesetzt werden kationische Cellulosederivate; kationische Stärke; Copolymere von Diallylammoniumsalzen und Acrylamiden; quaternierte Vinylpyrrolidon/ Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z.B. Amidomethicone; Copolymere der Adipinsäure und Dimethylaminohydroxy-propyldiethylentriamin; Polyaminopolyamid und kationische Chitinderivate, wie beispielsweise Chitosan.

Geeignete Siliconverbindungen sind beispielsweise Dimethylpolysiloxan, Methylphenylpolysiloxane, cyclische Silicone und amino-, fettsäure-, alkohol-, polyether-, epoxy-, fluor- und/oder alkylmodifizierte Siliconverbindungen, sowie Polyalkylsiloxane, Polyalkylarylsiloxane, Polyethersiloxan-Copolymere, wie in US 5,104,645 und den darin zitierten Schriften beschrieben, die bei Raumtemperatur sowohl flüssig als auch harzförmig vorliegen können.

Geeignete Filmbildner sind, je nach Anwendungszweck Salze der Phenylbenzimidazolsulfonsäure, wasserlösliche Polyurethane, beispielsweise C10-Polycarbamyl Polyglycerolester, Polyvinylalkohol, Polyvinylpyrrolidon, -copolymere, beispielsweise Vinylpyrrolidon/Vinylacetatcopolymer, wasserlösliche Acrylsäurepolymere/Copolymere bzw. deren Ester oder Salze, beispielsweise Partialestercopolymere der Acryl/ Methacrylsäure und Polyethylenglykolether von Fettalkoholen, wie Acrylat/Steareth-20-Methacrylat Copolymer, wasserlösliche Cellulose, beispielsweise Hydroxymethylcellulose, Hydroxyethylcellulose, Hydroxypropylcellulose, wasserlösliche Quaterniums, Polyquaterniums, Carboxyvinyl-Polymere, wie Carbomere und deren Salze, Polysaccharide, beispielsweise Polydextrose und Glucan.

Als Überfettungsmittel können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Als feuchtigkeitsspendende Substanz stehen beispielsweise Isopropylpalmitat, Glycerin und/oder Sorbitol zu Verfügung.

Als Stabilisatoren können Metallsalze von Fettsäuren, wie z.B. Magnesium-, Aluminium- und/oder Zinkstearat eingesetzt werden.

Unter biogenen Wirkstoffen sind beispielsweise Pflanzenextrakte und Vitaminkomplexe zu verstehen.

Zusätzlich können die erfindungsgemäßen Mittel organische Lösungsmittel enthalten. Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt. Weitere geeignete Lösungsmittel sind beispielsweise Triacetin (Glycerintriacetat) und 1-Methoxy-2-propanol. Hydrotrop wirken kurzkettige Aniontenside, insbesondere Arylsulfonate, beispielsweise Cumol- oder Toluolsulfonat.

Die erfindungsgemäßen Mittel können mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen als Pflegezusatz abgemischt werden.

Als Konservierungsmittel in Betracht kommen beispielsweise Phenoxyethanol, Parabene, Pentandiol, Octandiol, Benzoesäure, Salicylsäure oder Sorbinsäure.

Als Farbstoffe können die für kosmetische Zwecke geeigneten und zugelassenen Substanzen verwendet werden.

Als fungizide Wirkstoffe eignen sich bevorzugt Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole Fluconazole, Itraconazole, Terconazole, Naftifine und Terbinafine, Zn-Pyrithion und Octopirox (Clariant)

In einer weiteren bevorzugten Ausführungsform der Erfindung enthalten die Reinigungsmittel ein oder mehrere UV-Filter.

Als UV-Filter kommen vorzugsweise in Betracht 4-Aminobenzoesäure; 3-(4'-Trimethylammonium)benzyliden-boran-2-on-methylsulfat; 3,3,5-Trimethylcyclohexylsalicylat; 2-Hydroxy-4-methoxybenzophenon; 2-Phenylbenzimidazol-5-sulfonsäure und ihre Kalium-, Natrium- und Triethanolaminsalze; 3,3'-(1,4-Phenylendimethin)-bis-(7,7-dimethyl-2-oxobicyclo[2.2.1]-heptan-1-methansulfonsäure und ihre Salze; 1-(4-tert.-Butylphenyl)-3-(4-methoxyphenyl)propan-1,3-dion, 3-(4'-Sulfo)-benzyliden-bornan-2-on und seine Salze; 2-Cyan-3,3-diphenyl-Acrylsäure-(2-ethylhexylester); Polymer von N-[2(und 4)-(2-oxoborn-3-ylidenmethyl)benzyl]-acrylamid; 4-Methoxy-zimtsäure-2-ethylhexylester; ethoxyliertes Ethyl-4-amino-benzoat; 4-Methoxy-zimtsäure-isoamylester; 2,4,6-Tris-[p-(2-ethylhexyloxycarbonyl)anilino]-1,3,5-triazin; 2-(2N-benzotriazol-2-yl)-4-methyl-6-(2-methyl-3-(1,3,3,3-tetramethyl-1-(trimethylsilyloxy)-disiloxanyl)propyl)phenol; 4,4'-[(6-[4-((1,1-dimethylethyl)-aminocarbonyl)phenylamino]-1,3,5-triazin-2,4-yl)diimino]bis-(benzoesäure-2-ethylhexylester); 3-(4'-Methylbenzyliden)-D,L-Campher; 3-Benzyliden-Campher; Salicylsäure-2-ethylhexylester; 4-Dimethylaminobenzoesäure-2-ethylhexylester; Hydroxy-4-methoxy-benzophenon-5-sulfonsäure (Sulisobenzonum) und das Natriumsalz; und/oder 4-Isopropylbenzylsalicylat, N,N,N-Trimethyl-4-(2-oxoborn-3-ylidenemethyl)anilinium methyl sulfate, Homosalate (INN) Oxybenzone (INN) 2-Phenylbenzimidazole-5-sulfonsäure und ihre Na, K, und Triethanolaminsalze, alpha-(2-Oxoborn-3-ylidene) toluol-4-sulfonsäure und ihre Salze, Octylmethoxyzimtsäure, Isopentyl-4-methoxyzimtsäure, Isoamyl-p-methoxyzimtsäure, 2,4,6-Trianilino-(p-carbo-2'-ethylhexyl-1'-oxy)-1,3,5-triazine (Octyl triazone) Phenol, 2-2(2H-benzotriazol-2-yl)-4-methyl-6-(2-methyl-3-(1,3,3,3-tetramethyl-1-(trimethylsilyl)oxy)-disiloxanyl)propyl (Drometriazole Trisiloxane) benzoesäure, 4,4-((6-(((1,1-dimethylethyl)amino)carbonyl)phenyl)amino)-1,3,5-triazine-2,4-diyl)diimino)bis-, bis(2-ethylhexyl)ester) benzoesäure, 4,4-((6-(((1,1-dimethylethyl)amino)-carbonyl)phenyl)amino)-1,3,5-triazine-2,4-diyl)diimino)bis-, bis(2-ethylhexyl)ester) 3-(4'-Methylbenzylidene)-d,l-camphor (4-Methylbenzylidene Camphor) 3-Benzylidene camphor (3-Benzylidene camphor) 2-Ethylhexyl salicylat (Octyl Salicylat) 4-Dimethyl-aminobenzoat von ethyl-2-hexyl (octyl dimethyl PABA), 2-Hydroxy-4-methoxybenzophenone-5-sulfonsäure (Benzophenone-5) und das Na-Salz, 2,2'-Methylen-bis-6-(2H-benzotriazol-2yl)-4-(tetramethyl-butyl)-1,1,3,3-phenol, Natriumsalz von 2-2'-bis-(1,4-phenylen)1 H-benzimidazole-4,6-disulfonsäure, (1,3,5)-Triazine-2,4-bis((4-(2-ethylhexyloxy)-2-hydroxy)-phenyl)-6-(4-methoxyphenyl), 2-Ethylhexyl-2-cyano-3,3-diphenyl-2-propenoat, Glyceryl octanoat Di-p-methoxy zimtsäure, p-Amino-benzoesäure und Ester, 4-tert-Butyl-4'-methoxydibenzoylmethan, 4-(2-[beta]-glucopyranoxy)propoxy-2-hydroxybenzophenon, Octyl Salicylat, Methyl-2,5-diisopropylzimtsäure, Cinoxat, Dihydroxy-dimethoxybenzophenon, Dinatriumsalz von 2,2'-dihydroxy-4,4'-dimethoxy-5,5'-disulfobenzophenon, Dihydroxybenzophenon, 1-3,4-Dimethoxyphenyl)-4,4-dimethyl-1,3-pentanedion, 2-Ethylhexyl dimethoxybenzylidene dioxoimidazolidin propionat, Tetrahydroxybenzophenone, Terephthalidendicamphorsulfonsäure, 2,4,6-tris[4-2-ethylhexyloxycarbonyl)anilino]-1,3,5-triazin, Methyl bis(trimethylsiloxy)silyl isopentyltrimethoxy-zimtsäure, Amyl-p-dimethylamino benzoat, Amyl-p-dimethylamino benzoat, 2-Ethylhexyl- p-dimethylamino benzoat, Isopropyl-p-methoxyzimtsäure/Diisopropylzimtsäureester, 2-Ethylhexyl p-methoxyzimtsäure, 2-Hydroxy-4-methoxybenzophenon, 2-Hydroxy-4-methoxybenzophenon-5-sulfonsäure und das Trihydrat, 2-Hydroxy-4-methoxybenzophenon-5-sulfonat, Na-Salz, Phenyl benzimidazolsulfonsäure.

Die erfindungsgemässen Mittel enthalten UV-Lichtschutzfilter in den Mengen von vorzugsweise 0,1 bis 10 Gew.-%, besonders bevorzugt 0,5 bis 8 Gew.-% und insbesondere bevorzugt 1 bis 5 Gew.-%, bezogen auf das Gesamtgewicht der Mittel.

In einer weiteren bevorzugten Ausführungsform der Erfindung enthalten die Reinigungsmittel ein oder mehrere Antioxidantien.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen, ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid), Superoxid-Dismutase und die erfindungsgemäß geeigneten Derivate (Salze, Ester, Ether, Zucker, Nukleotide, Nukleoside, Peptide und Lipide) dieser genannten Stoffe.

Besonders vorteilhaft im Sinne der vorliegenden Erfindung können wasserlösliche Antioxidantien eingesetzt werden.

Die Antioxidantien können die Haut und das Haar vor oxidativer Beanspruchung schützen. Bevorzugte Antioxidantien sind dabei Vitamin E und dessen Derivate sowie Vitamin A und dessen Derivate.

Die Menge des einen oder der mehreren Antioxidantien in den erfindungsgemäßen Mitteln beträgt vorzugsweise von 0,001 bis 30 Gew.-%, besonders bevorzugt von 0,05 bis 20 Gew.-% und insbesondere bevorzugt von 1 bis 10 Gew.-%, bezogen auf das Gesamtgewicht der Mittel.

Sofern Vitamin E und/oder dessen Derivate das oder die Antioxidantien darstellen, ist es vorteilhaft, deren jeweilige Konzentrationen aus dem Bereich von 0,001 bis 10 Gew.-%, bezogen auf das Gesamtgewicht der Mittel, zu wählen.

Sofern Vitamin A, bzw. Vitamin-A-Derivate, bzw. Carotine bzw. deren Derivate das oder die Antioxidantien darstellen, ist es vorteilhaft, deren jeweilige Konzentrationen aus dem Bereich von 0,001 bis 10 Gew.-%, bezogen auf das Gesamtgewicht der Mittel, zu wählen.

In einer besonders bevorzugten Ausführungsform der Erfindung enthalten die erfindungsgemäßen Mittel Antioxidantien ausgewählt aus Superoxid-Dismutase, Tocopherol (Vitamin E) und Ascorbinsäure (Vitamin C).

Bei den erfindungsgemäßen Mitteln handelt es sich bevorzugt um Rinse-off-Formulierungen, insbesondere um Shampoos, Duschbäder, Duschgels, Schaumbäder und Flüssigseifen. Moderne Rinse-off Produkte haben häufig einen hohen Anteil an konditionierenden Wirkstoffen, die auch aus Ölanteilen bestehen können. Folglich können diese Mittel als Emulsionen vorliegen.

In einer besonders bevorzugten Ausführungsform der Erfindung handelt es sich bei den Reinigungsmitteln um Shampoos, Duschbäder oder Flüssigseifen.

Die nachfolgenden Beispiele sollen den Gegenstand der Erfindung näher erläutern, ohne ihn darauf einzuschränken (bei den Prozentangaben handelt es sich um Gew.-%). Viskositäten wurden mittels eine Brookfield-Viskosimeters (Spindel Nr. 3) gemessen.

### Beispiele

### Beispiel 1:

Haarshampoo, klar, 11,15 % Aktivtensidgehalt

| | | | |
|---|---|---|---|
| A | Hostapur^{®} SAS 60 | (Clariant) | 10.00 % |
| | sekundäres C₁₄₋₁₇ Alkylsulfonat, Na-Salz | | |
| | Lamesoft^{®} PO 65 | | 1.00 % |
| | Cocoglucosid und Glyceryloleat | | |
| | Duftstoff | | 0.30 % |
| B | Wasser | | ad 100.00 % |
| C | Genagen^{®} CAB | (Clariant) | 15.00 % |
| | Cocamidopropyl Betain | | |
| | Konservierungsmittel | | q.s. |
| D | Zitronensäure | | q.s. |

### Herstellung:

I Mischen der Komponenten A
II Zugabe von B zu I unter Rühren bis eine klare Lösung erhalten wird
III Zugabe von C zu II unter Rühren
IV Einstellen des pH auf 5.5 - 6.0 mit D

Viskosität Brookfield pH 5.7: 3600 mPas

### Vergleichsbeispiel 1:

Inhaltsstoffe und Herstellweise analog zu Beispiel 1. Das Gewichtsverhältnis von sekundärem Alkansulfonat zu Alkylamidopropylbetain bei gleichem Gesamtaktivgehalt wurde von 6 : 4,5 = 1,33 auf 7 : 3 = 2,33 geändert. Die Formulierung wies eine Viskosität von nur 70 mPas bei pH = 5.7 auf.

### Beispiel 2:

Haarshampoo für Männer, klar, 15,5 % Aktivtensidgehalt

| | | | |
|---|---|---|---|
| A | Hostapur^{®} SAS 60 | (Clariant) | 8.00 % |
| | sekundäres C₁₄₋₁₇ Alkylsulfonat, Na-Salz | | |
| | Hostapon^{®} CGN | (Clariant) | 10.00 % |
| | Cocoyl Glutamat, Na-Salz | | |
| | Plantacare 818 | | 3.00 % |
| | Coco Glucosid | | |
| | Rewomid IPP 240 | | 2.00 % |
| | Cocamid MIPA | | |
| | Wasser | | 20.00 % |
| B | Duftstoff | | 0.30 % |
| C | Wasser | | ad 100.00 % |
| D | Genagen^{®} CAB | (Clariant) | 8.00 % |
| | Cocamidopropyl Betain | | |
| | Genagen^{®} KB | (Clariant) | 6.00 % |
| | Coco Betain | | |
| | Konservierungsmittel | | q.s. |
| E | Zitronensäure | | q.s. |

### Herstellung:

I Lösen von A unter Rühren bei ca. 50°C
II Zugabe von B zu I unter Rühren bei ca. 35°C
III Zugabe von C zu II unter Rühren bis eine klare Lösung erhalten wird
IV Zugabe der Komponenten D zu III unter Rühren
V Einstellen auf pH 5,7 - 6,3 mit E

Viskosität Brookfield pH 6.3: 4430 mPas

### Beispiel 3:

Mildes Haarshampoo, klar, 15,5 % Aktivtensidgehalt

| | | | |
|---|---|---|---|
| A | Hostapur^{®} SAS 60 | (Clariant) | 8.00 % |
| | sekundäres C₁₄₋₁₇ Alkylsulfonat, Na-Salz | | |
| | Hostapon^{®} CGN | (Clariant) | 10.00 % |
| | Cocoyl Glutamat, Na-Salz | | |
| | Rewomid IPP 240 | | 2.00% |
| | Cocamid MIPA | | |
| | Plantacare 818 | | 3.00 % |
| | Coco Glucosid | | |
| | Wasser | | 20.00 % |
| B | Duftstoff | | 0.30 % |
| C | Wasser | | ad 100.00 % |
| | Glycerin | | 1.00 % |
| | Sorbitol | | 0.50 % |
| | Panthenol | | 1.00 % |
| D | Genagen^{®} CAB | (Clariant) | 8.00 % |
| | Cocamidopropyl Betain | | |
| | Genagen^{®} KB | (Clariant) | 6.00 % |
| | Coco Betain | | |
| E | Mackpro SLP | | 0.50 % |
| | Quaternium 79 hydrolysiertes | Soyaprotein | |
| | Konservierungsmittel | | q.s. |
| F | Zitronensäure | | q.s. |

### Herstellung:

I Lösen von A unter Rühren bei ca. 50°C
II Zugabe von B unter Rühren bei ca. 35°C
III Zugabe der Komponenten C zu II unter Rühren bis eine klare Lösung entstanden ist
IV Zugabe der Komponenten D in III
V Zugabe von E zu IV
VI Einstellen des pH auf 5,5 - 6,0 mit F

Viskosität Brookfield, pH 6.0 3720 mPas

### Beispiel 4:

Duschbad, klar, 16,75 % Aktivtensidgehalt

| | | | |
|---|---|---|---|
| A | Hostapur^{®} SAS 60 | (Clariant) | 10.00 % |
| | sekundäres C₁₄₋₁₇ Alkylsulfonat, Na-Salz | | |
| | Hostapon^{®} CGN | (Clariant) | 10.00 % |
| | Cocoyl Glutamat, Na-Salz | | |
| | Lamesoft^{®} PO 65 | | 1.00 % |
| | Coco Glucosid und Glyceryl Oleat | | |
| | Rewomid IPP 240 | | 1.00 % |
| | Cocamid MIPA | | |
| | Plantacare 818 | | 2.00 % |
| | Coco Glucosid | | |
| | Wasser | | 20.00 % |
| B | Duftstoff | | 0.50 % |
| C | Wasser | | ad 100.00 % |
| | Glycerin | | 1.00 % |
| | Sorbitol | | 1.00 % |
| | Avocado Spezial | | 1.00 % |
| | Wasser, Ethoxydiglycol, Propylen Glycol,Butylen Glycol,Persea Gratissima Extract | | |
| D | Genagen^{®} CAB | (Clariant) | 10.00 % |
| | Cocamidopropyl Betain | | |
| | Genagen^{®} KB | (Clariant) | 7.00 % |
| | Coco Betain | | |
| | Konservierungsmittel | | q.s. |
| E | Zitronensäure | | q.s. |

### Herstellung:

I Lösen von A unter Rühren bei ca. 50 °C
II Einrühren von B bei ca. 35 °C
III Zugabe der Komponenten C zu II und Rühren bis eine klare Lösung entstanden ist
IV Zugabe der Komponenten D in III
V Einstellen des pH auf 5.5 - 6.0 mit E

Viskosität Brookfield; pH 6.0 4120 mPas

INCI-Liste und Konzentrationen der Inhaltsstoffe. Die Konzentrationsangaben in dieser Anmeldung sind jeweils auf den Aktivgehalt der Tenside bezogen.

| | |
|---|---|
| Genagen^{®} CAB | Cocamidopropylbetain, 30 % Lösung |
| Genagen^{®} KB | Coco Betaine, 30 % Lösung |
| Hostapur^{®} SAS 60 | sekundäres C₁₄₋₁₇ Alkylsulfonat, 60 % Paste |
| Plantacare 818 | Coco Glucoside, 50 % wäßr. Lösung |
| Rewomid IPP 240 | Cocamide MIPA, 100 % aktiv |
| Lamesoft^{®} PO 65 | Coco Glucoside und Glyceryl Oleat, 65 % Lösung |
| Hostapon^{®} CGN | Sodium Cocoyl Glutamate, 30 % Lösung |
| Mackpro SLP | Quaternium 79 |

## Patentansprüche

1. Stabiles flüssiges kosmetisches oder dermatologisches Reinigungsmittel enthaltend ein oder mehrere sekundäre Alkansulfonate und ein oder mehrere Betaine, **dadurch gekennzeichnet, dass** das Gewichtsverhältnis von Alkansulfonat zu Betain von 6 : 3 bis 1: 2 ist und die Viskosität des Reinigungsmittels zwischen 1000 und 30000 mPas beträgt.

2. Reinigungsmittel nach Anspruch 1, **dadurch gekennzeichnet, dass** das Gewichtsverhältnis von Alkansulfonat zu Betain von 4 : 3 bis 1 : 1 ist.

3. Reinigungsmittel nach Anspruch 1 oder 2, **dadurch gekennzeichnet, dass** die Viskosität zwischen 2000 und 10000 mPas beträgt.

4. Reinigungsmittel nach einem oder mehreren der Ansprüche 1 bis 3, **dadurch gekennzeichnet, dass** die Menge an dem einen oder den mehreren sekundären Alkansulfonaten und dem einen oder den mehreren Betainen zusammen zwischen 6 und 20 Gew.-%, bezogen auf das Gesamtgewicht des Mittels, beträgt.

5. Reinigungsmittel nach Anspruch 4, **dadurch gekennzeichnet, dass** die Menge an dem einen oder den mehreren sekundären Alkansulfonaten und dem einen oder den mehreren Betainen zusammen zwischen 8 und 15 Gew.-%, bezogen auf das Gesamtgewicht des Mittels, beträgt.

6. Reinigungsmittel nach einem oder mehreren der Ansprüche 1 bis 5, **dadurch gekennzeichnet, dass** das eine oder die mehreren Betaine ausgewählt sind aus der Gruppe bestehend aus Alkylamidopropylbetainen mit 8 bis 18 Kohlenstoffatomen in der Alkylgruppe des Fettsäurerests, Alkylbetainen mit 8 bis 18 Kohlenstoffen in der Alkylgruppe und Mischungen aus diesen Substanzen.

7. Reinigungsmittel nach Anspruch 6, **dadurch gekennzeichnet, dass** das eine oder die mehreren Betaine ausgewählt sind aus Alkylamidopropylbetain oder einer Mischung aus Alkylamidopropylbetain und Alkylbetain.

8. Reinigungsmittel nach einem oder mehreren der Ansprüche 1 bis 7, **dadurch gekennzeichnet, dass** es klar ist.

9. Reinigungsmittel nach einem oder mehreren der Ansprüche 1 bis 8, **dadurch gekennzeichnet, dass** das eine oder die mehreren sekundären Alkansulfonate ausgewählt sind aus C₈₋₂₀ Alkansufonaten.

10. Reinigungsmittel nach Anspruch 9, **dadurch gekennzeichnet, dass** das eine oder die mehreren sekundären Alkansulfonate ausgewählt sind aus C₁₄₋₁₇ Alkansulfonaten, vorzugsweise aus deren Natriumsalzen.

11. Reinigungsmittel nach einem oder mehreren der Ansprüche 1 bis 10, **dadurch gekennzeichnet, dass** es keine ethoxylierten oder propoxylierten Verbindungen enthält.

12. Reinigungsmittel nach einem oder mehreren der Ansprüche 1 bis 11, **dadurch gekennzeichnet, dass** es keine Alkylethersulfate enthält.

13. Reinigungsmittel nach einem oder mehreren der Ansprüche 1 bis 12, **dadurch gekennzeichnet, dass** es ein oder mehrere Cotenside enthält.

14. Reinigungsmittel nach Anspruch 13, **dadurch gekennzeichnet, dass** das eine oder die mehreren Cotenside ausgewählt sind aus Acylglutamaten, Alkylpolyglucosiden, Amphoacetaten, Cocosmonoethanolamiden, Cocosisopropanolamiden oder Mischungen aus diesen Substanzen.

15. Reinigungsmittel nach Anspruch 13 oder 14, **dadurch gekennzeichnet, dass** die Gesamtmenge an Tensiden, bezogen auf das Gesamtgewicht des Mittels, von 6,5 bis 20 Gew.-% beträgt.

16. Reinigungsmittel nach einem oder mehreren der Ansprüche 1 bis 15, **dadurch gekennzeichnet, dass** es sich um ein Shampoo, Duschbad oder eine Flüssigseife handelt.
